# EUROPEAN PATENT APPLICATION

(11) **EP 3 656 824 A1**
(43) Date of publication of application: **27.05.2020**
(21) Application number: 18208207.3
(22) Date of filing: 26.11.2018
(51) Int. Cl.: C09D 11/101, C09D 11/38, C07F 9/53, C07D 335/00, C08K 5/5397

(54) **RADIATION CURABLE INKJET FOR MANUFACTURING PRINTED CIRCUIT BOARDS**

(71) Applicant: AGFA-GEVAERT NV, 2640 Mortsel (BE)
(72) Inventor: LOCCUFIER, Johan, 2640 Mortsel (BE); TORFS, Rita, 2640 Mortsel (BE); SAUVAGEOT, Marion, 2640 Mortsel (BE)
(74) Representative: Viaene, Kris

(57) **Abstract**

A radiation curable inkjet ink comprising a polymerizable compound and a photoinitiator, characterized in that the photoinitiator comprises a functional group selected from the group consisting of an aliphatic thio-ether and an aliphatic or a (hetero)aromatic disulfide.

## Description

### Technical field of the invention

The present invention relates to radiation curable inkjet inks and inkjet methods for manufacturing Printed Circuit Boards.

### Background art for the invention

The production workflow of printed circuit boards (PCBs) is gradually shifting from the standard workflow towards a digital workflow to reduce the amount of process steps and to lower the cost and the environmental impact of the production of PCBs, especially for short run productions.

Inkjet is one of the preferred digital manufacturing technologies in different steps of the PCB manufacturing process going from etch resist over solder mask to legend printing. Preferred inkjet inks are UV curable ink jet inks.

Inkjet printing methods and inkjet inks have been disclosed for legend printing in for example EP-A 2725075 (AGFA) and US 7845785 (MARKEM-IMAJE) and for applying an etch resist on a copper surface in for example EP-A 2809735 (AGFA) and EP-A 3000853 (AGFA).

Also for applying the solder mask, inkjet printing methods and inkjet inks have been disclosed in for example EP-A 1543704 (AVECIA) and EP-A 1624001 (TAIYO INK MANUFACTURING).

In the different production steps, adhesion of the inkjet inks towards different substrates is of crucial importance. To maximize the adhesion performance, adhesion promoters are often required.

Several classes of adhesion promoters have been disclosed in the prior art, most of them acidic in nature.

WO2004/026977 (AVECIA) discloses a non-aqueous etch resistant inkjet ink comprising 1 to 30 wt% of an acrylate functional monomer containing one or more acidic group as an adhesion promoter and dissolution promoter during stripping.

WO2004/106437 (AVECIA) discloses an etch resistant inkjet ink preferably comprising (meth)acrylate acid adhesion promoters, such as (meth)acrylated carboxylic acids, (meth)acrylated phosphoric acid esters and (meth)acrylated sulphonic acids.

In pigmented systems, such as solder mask inks and legend inks, acidic adhesion promoters often interfere with the pigment dispersions, leading to a limited stability of the inks. This may have an impact on the reliability of industrial printing systems in PCB printing, leading to an unacceptable loss in productivity.

Therefore, there is still a need to design jettable radiation curable formulations without the need for acidic adhesion promoters.

### Summary of the invention

It is an object of the invention to provide a radiation curable inkjet ink for use in a PCB manufacturing process characterized by a good adhesion to various substrates while maintaining excellent jetting and stability performance.

That object of the invention is realized by the radiation curable inkjet ink according to claim 1.

It has been found that a radiation curable composition comprising a photoinitiator as defined in claim 1 has an excellent adhesion towards various substrates without the need for acidic adhesion promoters.

Further objects of the invention will become apparent from the description hereinafter.

### Detailed description of the invention

### Definitions

The term "monofunctional" in e.g. monofunctional polymerizable compound means that the polymerizable compound includes one polymerizable group.

The term "difunctional" in e.g. difunctional polymerizable compound means that the polymerizable compound includes two polymerizable groups.

The term "polyfunctional" in e.g. polyfunctional polymerizable compound means that the polymerizable compound includes more than two polymerizable groups.

The term "alkyl" means all variants possible for each number of carbon atoms in the alkyl group i.e. methyl, ethyl, for three carbon atoms: n-propyl and isopropyl; for four carbon atoms: n-butyl, isobutyl and tertiary-butyl; for five carbon atoms: n-pentyl, 1,1-dimethyl-propyl, 2,2-dimethylpropyl and 2-methyl-butyl, etc.

Unless otherwise specified a substituted or unsubstituted alkyl group is preferably a C₁ to C₆-alkyl group.

Unless otherwise specified a substituted or unsubstituted alkenyl group is preferably a C₂ to C₆-alkenyl group.

Unless otherwise specified a substituted or unsubstituted alkynyl group is preferably a C₂ to C₆-alkynyl group.

Unless otherwise specified a substituted or unsubstituted alkaryl group is preferably a phenyl or naphthyl group including one, two, three or more C₁ to C₆-alkyl groups.

Unless otherwise specified a substituted or unsubstituted aralkyl group is preferably a C₇ to C₂₀-alkyl group including a phenyl group or naphthyl group.

Unless otherwise specified a substituted or unsubstituted aryl group is preferably a phenyl group or naphthyl group.

Unless otherwise specified a substituted or unsubstituted heteroaryl group is preferably a five- or six-membered ring substituted by one, two or three oxygen atoms, nitrogen atoms, sulphur atoms, selenium atoms or combinations thereof.

The term "substituted", in e.g. substituted alkyl group means that the alkyl group may be substituted by other atoms than the atoms normally present in such a group, i.e. carbon and hydrogen. For example, a substituted alkyl group may include a halogen atom or a thiol group. An unsubstituted alkyl group contains only carbon and hydrogen atoms.

Unless otherwise specified a substituted alkyl group, a substituted alkenyl group, a substituted alkynyl group, a substituted aralkyl group, a substituted alkaryl group, a substituted aryl and a substituted heteroaryl group are preferably substituted by one or more constituents selected from the group consisting of methyl, ethyl, n-propyl, isopropyl, n-butyl, isobutyl and tertiary-butyl, ester, amide, ether, thioether, ketone, aldehyde, sulfoxide, sulfone, sulfonate ester, sulphonamide, -CI, -Br, -I, -OH, -SH, - CN and -NO₂.

### Radiation curable inkjet ink

The radiation curable inkjet ink comprises a polymerizable compound and photoinitiator as described below.

The radiation curable inkjet ink may further comprise colorants, polymeric dispersants, a polymerization inhibitor, a flame retardant or a surfactant.

The radiation curable inkjet ink may be cured by any type of radiation, for example by electron-beam radiation, but is preferably cured by UV radiation, more preferably by UV radiation from UV LEDs. The radiation curable inkjet ink is thus preferably a UV curable inkjet ink.

For reliable industrial inkjet printing, the viscosity of the radiation curable inkjet ink is preferably no more than 20 mPa.s at 45°C, more preferably between 1 and 18 mPa.s at 45°C, and most preferably between 4 and 14 mPa.s at 45°C, all at a shear rate of 1000 s⁻¹.

A preferred jetting temperature is between 10 and 70°C, more preferably between 20 and 55°C, and most preferably between 25 and 50°C.

For good image quality and adhesion, the surface tension of the radiation curable inkjet ink is preferably in the range of 18 to 70 mN/m at 25°C, more preferably in the range of 20 to 40 mN/m at 25°C.

### Photoinitiator

The photoinitiator includes a functional group selected from the group consisting of an aliphatic thio-ether and an aliphatic or a (hetero)aromatic disulfide.

The photoinitiator is preferably selected from the group consisting of a thioxanthone, an α-hydroxyketone, an α-aminoketone, an acylphosphine oxide and a bisacyl phosphine oxide. The photoinitiator is more preferably an acylphosphine oxide or a bisacyl phosphine oxide.

In a preferred embodiment, the photoinitiator has a structure according to Formula I, 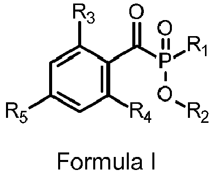 wherein
R₁ is selected from the group consisting of a substituted or unsubstituted aryl group, a substituted or unsubstituted heteroaryl group and an acyl group;
R₂ represents a structural moiety comprising a functional group selected from the group consisting of an aliphatic thio-ether and a disulfide;
R₃ and R₄ are independently selected from the group consisting of a substituted or unsubstituted alkyl group, a substituted or unsubstituted alkoxy group and a halogen;
R₅ is selected from the group consisting of a hydrogen, a substituted or unsubstituted alkyl group, a substituted or unsubstituted alkoxy group and a halogen.

R₁ preferably represents a substituted or unsubstituted aryl group, a phenyl group being most preferred.

Preferably, R₃ and R₄ are independently selected from a methyl group, a methoxy group and a chlorine atom.

R₃, R₄ and R₅ most preferably represent a methyl group.

In another preferred embodiment, the photoinitiator has a structure according to Formula II, 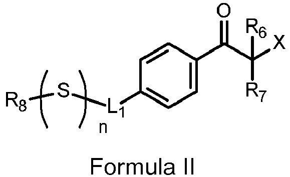 wherein
X is selected from the group consisting of OH and NR₉R₁₀;
R₆ and R₇ are independently selected from the group consisting of a substituted or unsubstituted alkyl group, a substituted or unsubstituted alkenyl group, a substituted or unsubstituted alkynyl group, a substituted or unsubstituted alkaryl group, a substituted or unsubstituted aralkyl group and a substituted or unsubstituted (hetero)aryl group;
R₆ and R₇ may represent the necessary atoms to form a five to eight membered ring;
R₈ is selected from the group consisting of a substituted or unsubstituted alkyl group, a substituted or unsubstituted alkenyl group, a substituted or unsubstituted alkynyl group and a substituted or unsubstituted aralkyl group;
L₁ is a divalent linking group having no more than 20 carbon atoms;
R₉ and R₁₀ are independently selected from the group consisting of a substituted or unsubstituted alkyl group, a substituted or unsubstituted alkenyl group, a substituted or unsubstituted alkynyl group, a substituted or unsubstituted alkaryl group, a substituted or unsubstituted aralkyl group and a substituted or unsubstituted (hetero)aryl group;
R₉ and R₁₀ may represent the necessary atoms to form a five to eight membered ring, preferably a five to six membered ring;
n represents 1 or 2.

Preferably, R₆ and R₇ independently represent a substituted or unsubstituted alkyl group and a substituted or unsubstituted aralkyl group, a C₁ to C₄ group and a substituted or unsubstituted benzyl group being particularly preferred.

Preferably, R₉ and R₁₀ independently represent a substituted or unsubstituted alkyl group. In another preferred embodiment.

L₁ preferably represents a divalent linking group containing no more than 10 carbon atoms, no more than six carbon atoms being particularly preferred.

In a particularly preferred embodiment, the photoinitiator has a structure according to Formula III, 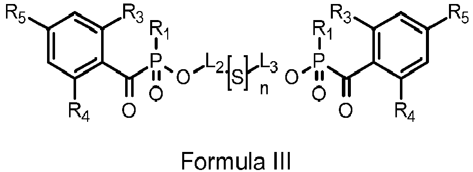 wherein
R₁, R₃, R₄ and R₅ are as defined above;
L₂ and L₃ independently represent a divalent linking groups having no more than 20 carbon atoms;
n represents 1 or 2.

Preferably, L₂ and L₃ independently represent a divalent linking group containing no more than 10 carbon atoms, no more than six carbon atoms being particularly preferred.

In a most preferred embodiment, the photoinitiator has a structure according to Formula IV, 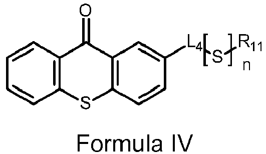 wherein
L₄ is a divalent linking group having no more than 20 carbon atoms;
R₁₁ is selected from the group consisting of a substituted or unsubstituted alkyl group, a substituted or unsubstituted alkenyl group, a substituted or unsubstituted alkynyl group and a substituted or unsubstituted aralkyl group;
n represents 1 or 2.

L₄ preferably represents a divalent linking group containing no more than 10 carbon atoms.

The photoinitiator according to Formula IV most preferably comprises at least two aliphatic thio-ethers.

The concentration of the photoinitiator is preferably from 0.5 to 15 wt%, more preferably from 1 to 10 wt%; most preferably from 1.5 to 6 wt%, all relative to the total weight of the inkjet ink.

Typical examples of photoinitiators according to the present invention are given in Table 1 below without being limited thereto.

**Table 1**

| | |
|---|---|
| 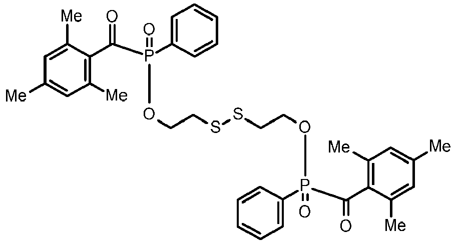 | INI-1 |
| 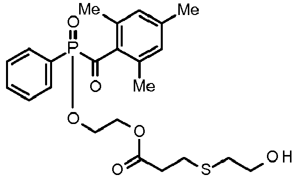 | INI-2 |
| 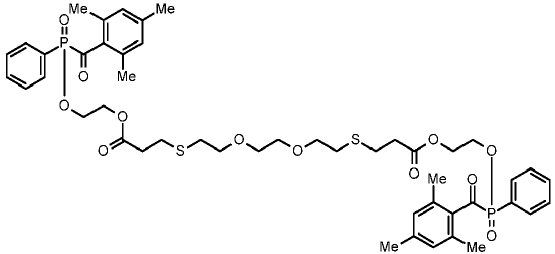 | INI-3 |
| 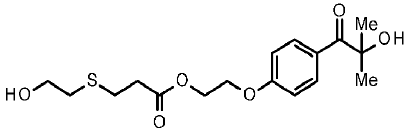 | INI-4 |
| 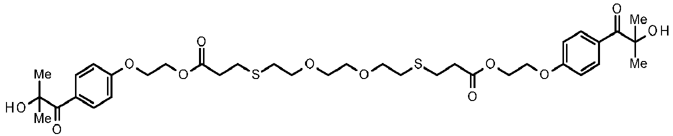 | INI-5 |
| 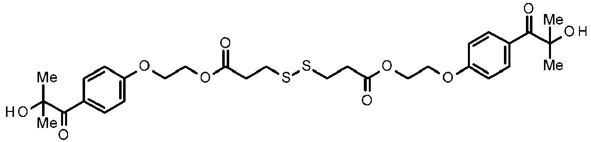 | INI-6 |
| 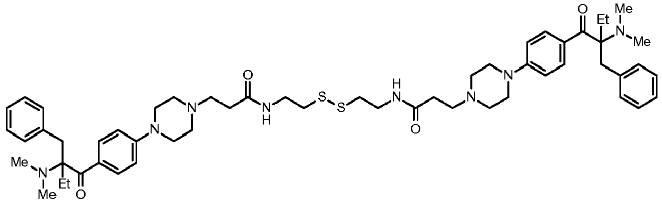 | INI-7 |
| 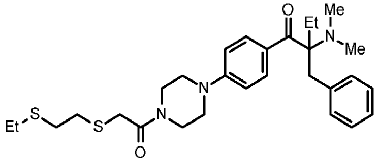 | INI-8 |
| 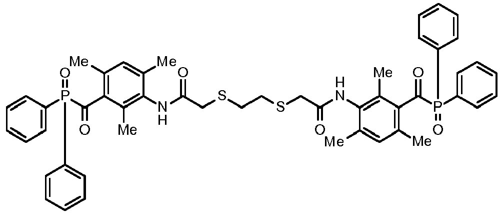 | INI-9 |
| 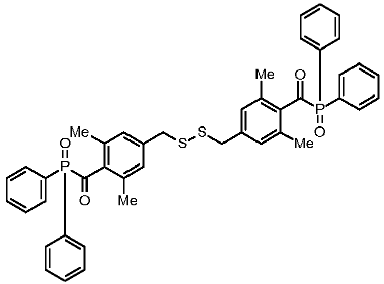 | INI-10 |
| 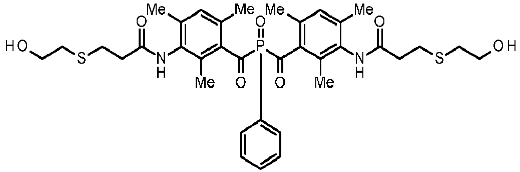 | INI-11 |
| 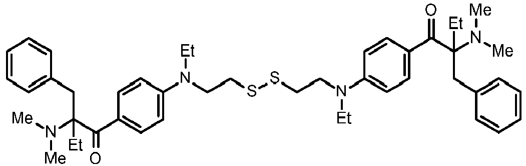 | INI-12 |
| 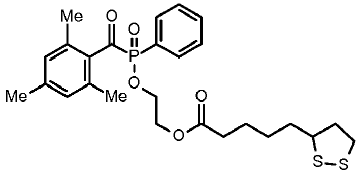 | INI-13 |
| 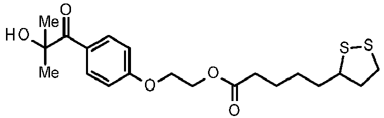 | INI-14 |
| 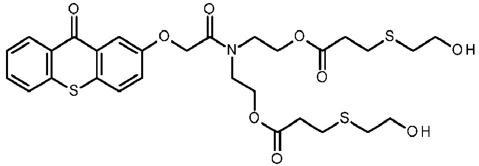 | INI-15 |
| 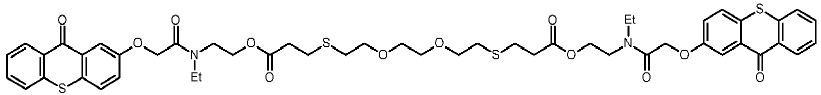 | INI-16 |
| 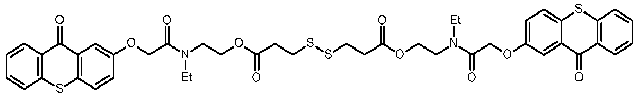 | INI-17 |
| 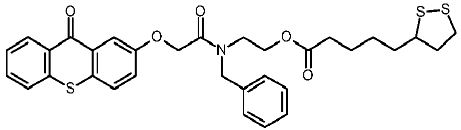 | INI-18 |

### Polymerizable compounds

The polymerizable compounds are preferably free radical polymerizable compounds.

The free radical polymerizable compounds may be monomers, oligomers and/or prepolymers. Monomers are also referred to as diluents.

These monomers, oligomers and/or prepolymers may possess different degrees of functionality, i.e. a different amount of free radical polymerizable groups.

A mixture including combinations of mono-, di-, tri-and higher functional monomers, oligomers and/or prepolymers may be used. The viscosity of the radiation curable inkjet ink may be adjusted by varying the ratio between the monomers and oligomers.

In a preferred embodiment, the monomer, oligomer or polymer includes at least one acrylate group as polymerizable group.

Preferred monomers and oligomers are those listed in paragraphs [0106] to [0115] in EP-A 1911814.

In a preferred embodiment, the radiation curable inkjet ink comprises a monomer containing a vinyl ether group and an acrylate or methacrylate group. Such monomers are disclosed in EP-A 2848659, paragraphs [0099] to [0104]). A particular preferred monomer containing a vinyl ether group and an acrylate group is 2-(2-vinyloxyethoxy)ethyl acrylate.

When used for forming a solder mask, the polymerizable compound is preferably selected from the group consisting of acryloyl morpholine, cyclic trimethyl propene formol acrylate, isobornyl acrylate, dipropylene glycol diacrylate, trimethylol propane triacrylate, and 2-(vinylethoxy)ethyl acrylate.

When the radiation curable inkjet ink is used for forming an etch resist, preferred polymerizable compounds are disclosed in WO2013/113572, paragraphs [0056] to [0058]; WO2015/132020, paragraphs [0031] to [0052]; or WO2016/050504, paragraph [0028] to [0066].

### Phenolic compound

The radiation curable inkjet ink preferably comprises a phenolic compound, more preferably a phenolic compound comprising at least two phenolic groups. The phenolic compound may comprise two, three, four or more phenolic groups.

A preferred phenolic compound comprises two phenolic groups.

A particular preferred phenolic compound has a structure according to Formula II: 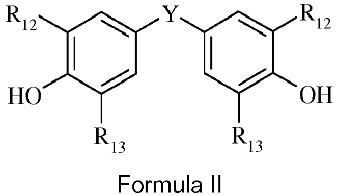 wherein,
R₁₂ and R₁₃ are independently selected from the group consisting of a hydrogen atom, a substituted or unsubstituted alkyl group, a hydroxyl group and a substituted or unsubstituted alkoxy group,
Y is selected from the group consisting of CR₁₄R₁₅, SO₂, SO, S, O and CO,
R₁₄ and R₁₅ are independently selected from the group consisting of a hydrogen atom, a substituted or unsubstituted alkyl group, a substituted or unsubstituted alkenyl group, a substituted or unsubstituted alkynyl group, a substituted or unsubstituted alkaryl group, a substituted or unsubstituted aralkyl group, a substituted or unsubstituted (hetero)aryl group,
R₁₄ and R₁₅ may represent the necessary atoms to form a 5 to 8 membered ring.

Y is preferably CR₁₄R₁₅ or SO₂, R₁₄ an R₁₅ preferably represent a hydrogen atom or an alkyl group.

In another preferred embodiment, the phenolic compound is a polymer comprising at least two phenolic groups. Preferably, the polymer comprising at least two phenolic groups is a branched or hyperbranched polymer.

A preferred polymer comprising at least two phenolic groups is a phenolic resin, i.e. a novolac or a resole.

Phenolic resins are reaction products of phenolic compounds with aldehydes or ketones. Phenols which could be used are: phenol, o-cresol, p-cresol, m-cresol, 2,4-xylenol, 3,5-xylenol, or 2,5-xylenol. Aldehydes which can be used are formaldehyde, acetaldehyde, or acetone.

The most widely used method for novolac preparation is the acid-catalysed one-step synthesis of phenol/cresol and formaldehyde, which leads to a statistical structure of resin (see reaction scheme below).

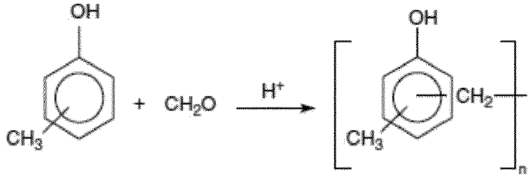

Generally, hydrochloric acid, sulfuric acid, p-toluene sulfuric acid or oxalic acid is used as catalyst. Various proportions of formaldehyde and phenol/cresol are usually employed in regular novolac resins. Higher phenol contents increase the degree of branching whereas reaction can take place at the ortho and para-positions. For resins with a higher p-cresol content more linear polymers are obtained due to that the para-position is blocked by presence of the methyl group.

Novolac copolymers of phenol and formaldehyde will have a high degree of branching, since reaction takes place both an ortho- and para-positions. In order to reduce the viscosity a high degree of branching and/or low molecular weights are preferred. For cresylic novolacs the use of m-cresol can give easier high molecular weights as compared to o-cresol and p-cresol.

Phenolic resins can also be prepared in base catalyzed reactions, which lead to the formation of resoles. Resoles are phenolic polymers having also methylol groups.

For incorporation in the solder mask inkjet ink, preference is given to novolac resins to obtain a sufficient ink stability since novolac resins are only reactive at high temperatures (>150 C). Resoles may react already at lower temperatures and due to the presence of methylol groups may result in a poorer chemical resistance of the inkjet ink.

More well defined branched polymers having at least two phenolic groups may be prepared using 4-hydroxyphenylmethylcarbinol, as disclosed in US5554719 and US2005250042. A particular preferred branched polymer having at least two phenolic groups prepared from 4-hydroxyphenylmethylcarbinol has been developed by Du Pont Electronic Polymers and is supplied by Hydrite Chemical Company under the tradename PB-5 (CASRN 166164-76-7).

Examples of phenolic compounds according to the present invention are given in Table 2 without being limited thereto.

**Table 2**

| | |
|---|---|
| 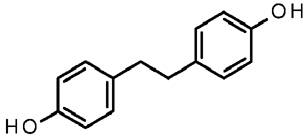 | PHEN-1 |
| 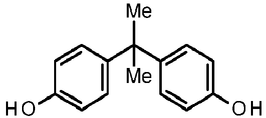 | PHEN-2 |
| 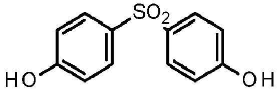 | PHEN-3 |
| 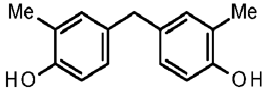 | PHEN-4 |
| 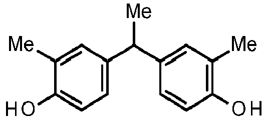 | PHEN-5 |
| 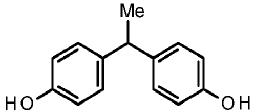 | PHEN-6 |
| 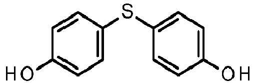 | PHEN-7 |
| 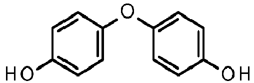 | PHEN-8 |
| 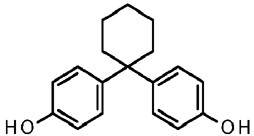 | PHEN-9 |
| 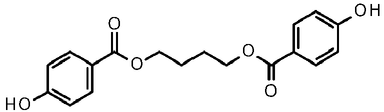 | PHEN-10 |
| 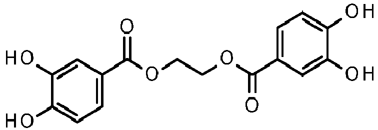 | PHEN-11 |
| 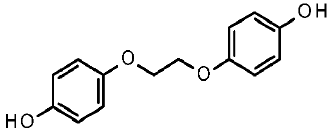 | PHEN-12 |
| 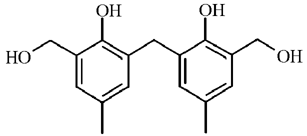 | PHEN-12 |
| 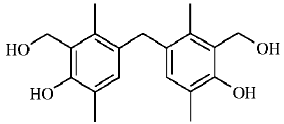 | PHEN-13 |
| 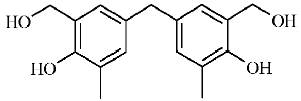 | PHEN-14 |
| 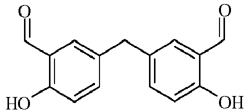 | PHEN-15 |
| 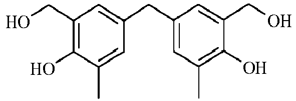 | PHEN-16 |
| 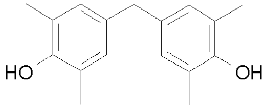 | PHEN-17 |
| 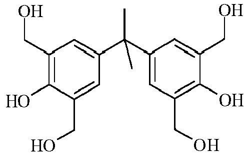 | PHEN-18 |
| 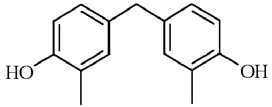 | PHEN-19 |
| 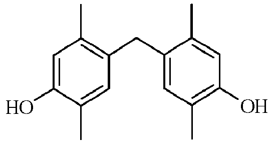 | PHEN-20 |
| 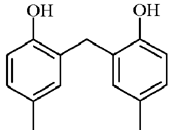 | PHEN-21 |
| | |
| 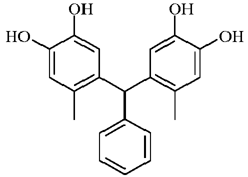 | PHEN-22 |
| 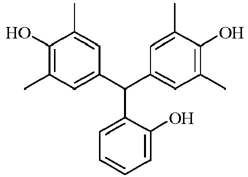 | PHEN-23 |
| 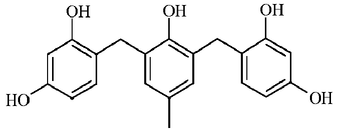 | PHEN-24 |
| 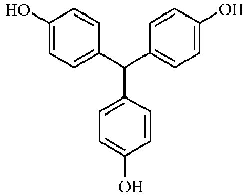 | PHEN-25 |
| 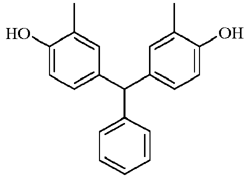 | PHEN-26 |
| 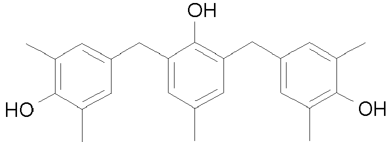 | PHEN-27 |
| 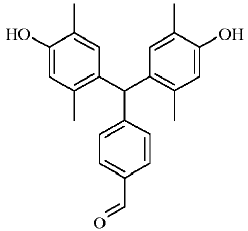 | PHEN -28 |
| 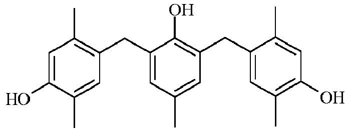 | PHEN-29 |
| 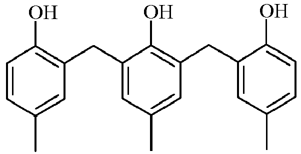 | PHEN-30 |
| 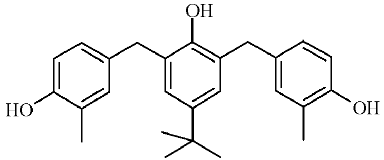 | PHEN-31 |
| | |
| 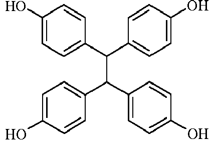 | PHEN-32 |
| 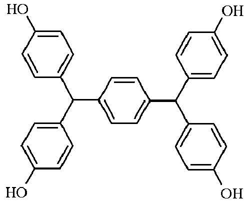 | PHEN-33 |
| 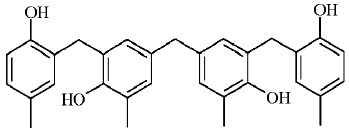 | PHEN-34 |
| 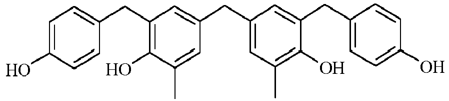 | PHEN-35 |
| 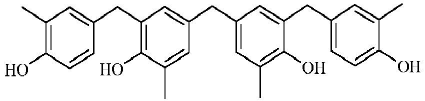 | PHEN-36 |
| 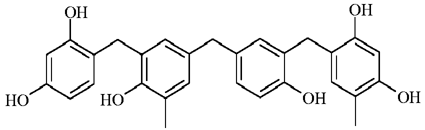 | PHEN-37 |
| 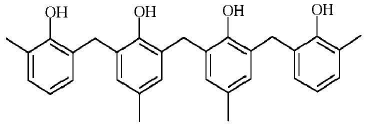 | PHEN-38 |
| 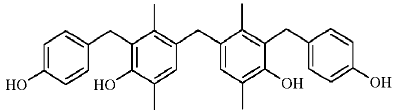 | PHEN-39 |
| 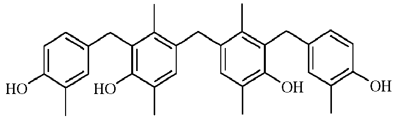 | PHEN-40 |
| 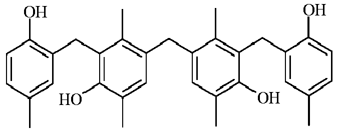 | PHEN-41 |
| 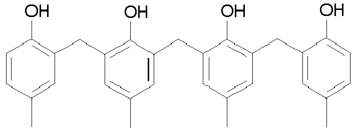 | PHEN-42 |

Typical examples of polymers having at least two phenolic groups are given in Table 3 below without being limited thereto.

**Table 3**

| | |
|---|---|
| 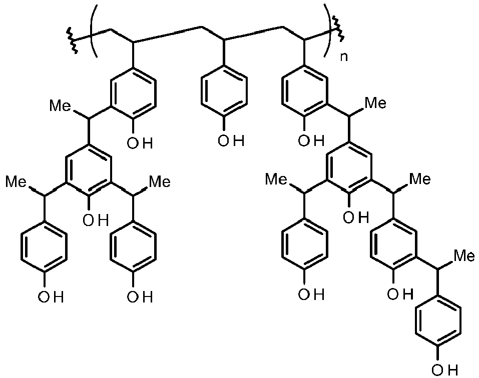 | RESIN-1 |
| 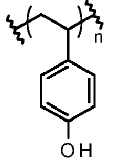 | RESIN-2 |
| 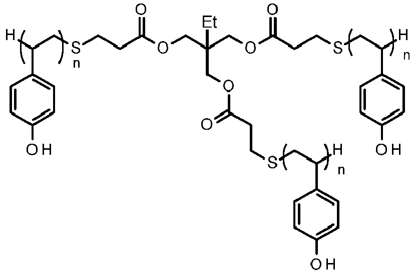 | RESIN-3 |
| 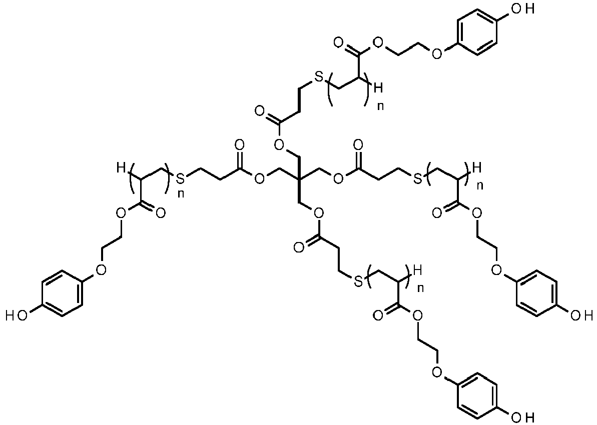 | RESIN -4 |

The amount of phenolic compounds is preferably between 0.5 and 20 wt%, more preferably between 1 and 15 wt%, most preferably between 2.5 and 10 wt%, relative to the total weight of the inkjet ink.

### Colorants

The radiation curable inkjet may be a substantially colourless inkjet ink or may include at least one colorant. For example when the inkjet ink is used as etch resist, the colorant makes the temporary mask clearly visible to the manufacturer of conductive patters, allowing a visual inspection of quality. When the inkjet ink is used to apply a solder mask it typically contains a colorant. A preferred colour for a solder mask is green, however other colours such as black or red may also be used.

The colorant may be a pigment or a dye, but is preferably a pigment.

A colour pigment may be chosen from those disclosed by HERBST, Willy, et al. Industrial Organic Pigments, Production, Properties, Applications, 3rd edition. Wiley - VCH, 2004, ISBN 3527305769.

Suitable pigments are disclosed in paragraphs [0128] to [0138] of WO2008/074548.

Pigment particles in inkjet inks should be sufficiently small to permit free flow of the ink through the inkjet-printing device, especially at the ejecting nozzles. It is also desirable to use small particles for maximum colour strength and to slow down sedimentation. Most preferably, the average pigment particle size is no larger than 150 nm. The average particle size of pigment particles is preferably determined with a Brookhaven Instruments Particle Sizer BI90plus based upon the principle of dynamic light scattering.

In PCBs, the solder mask typically has a blue or green colour. The blue pigment is preferably one of the phthalocyanine series. Examples of blue pigments are C.I. Pigment Blue 1, 15, 15:1, 15:2, 15:3, 15:4, 15:6, 16, 24 and 60.

Green pigments are generally a mixture of blue and yellow or orange pigments or may be green pigments or dyes per se, such as halogenated phthalocyanines, for example copper or nickel brominated phthalocyanine.

In a preferred embodiment, the colorant is present in an amount of 0.2 to 6.0 wt%, more preferably 0.5 to 2.5 wt%, based on the total weight of the radiation curable inkjet ink.

### Polymeric Dispersants

If the colorant in the radiation curable inkjet is a pigment, then the radiation curable inkjet ink preferably contains a dispersant, more preferably a polymeric dispersant, for dispersing the pigment.

Suitable polymeric dispersants are copolymers of two monomers but they may contain three, four, five or even more monomers. The properties of polymeric dispersants depend on both the nature of the monomers and their distribution in the polymer. Copolymeric dispersants preferably have the following polymer compositions:
- statistically polymerized monomers (e.g. monomers A and B polymerized into ABBAABAB);
- alternating polymerized monomers (e.g. monomers A and B polymerized into ABABABAB);
- gradient (tapered) polymerized monomers (e.g. monomers A and B polymerized into AAABAABBABBB);
- block copolymers (e.g. monomers A and B polymerized into AAAAABBBBBB) wherein the block length of each of the blocks (2, 3, 4, 5 or even more) is important for the dispersion capability of the polymeric dispersant;
- graft copolymers (graft copolymers consist of a polymeric backbone with polymeric side chains attached to the backbone); and
- mixed forms of these polymers, e.g. blocky gradient copolymers.

Suitable polymeric dispersants are listed in the section on "Dispersants", more specifically [0064] to [0070] and [0074] to [0077], in EP-A 1911814.

Commercial examples of polymeric dispersants are the following:
- DISPERBYK™ dispersants available from BYK CHEMIE GMBH;
- SOLSPERSE™ dispersants available from NOVEON;
- TEGO™ DISPERS™ dispersants from EVONIK;
- EDAPLAN™ dispersants from MÜNZING CHEMIE;
- ETHACRYL™ dispersants from LYONDELL;
- GANEX™ dispersants from ISP;
- DISPEX™ and EFKA™ dispersants from CIBA SPECIALTY CHEMICALS INC;
- DISPONER™ dispersants from DEUCHEM; and
- JONCRYL™ dispersants from JOHNSON POLYMER.

### Photoinitiators and Photoinitiating Systems

The radiation curable inkjet contains a photoinitiator as described above. However, the inkjet ink may contain other photoinitiators.

Often, a so-called co-initiator is used in combination with a photoinitiator. Such a combination of a co-initiator and a photoinitiator is referred to as photoinitiating systems.

Suitable photoinitiators are disclosed in CRIVELLO, J.V., et al. Photoinitiators for Free Radical Cationic and Anionic Photopolymerization. 2nd edition. Edited by BRADLEY, G.. London, UK: John Wiley and Sons Ltd, 1998. p.276-293.

In order to increase the photosensitivity further, the radiation curable inkjet ink may additionally contain co-initiators.

Suitable examples of co-initiators can be categorized in three groups:
(1) tertiary aliphatic amines such as methyldiethanolamine, dimethylethanolamine, triethanolamine, triethylamine and N-methylmorpholine;
(2) aromatic amines such as amylparadimethyl-aminobenzoate, 2-n-butoxyethyl-4-(dimethylamino) benzoate, 2-(dimethylamino)-ethylbenzoate, ethyl-4-(dimethylamino)benzoate, and 2-ethylhexyl-4-(dimethylamino)benzoate; and
(3) (meth)acrylated amines such as dialkylamino alkyl(meth)acrylates (e.g., diethylaminoethylacrylate) or N-morpholinoalkyl-(meth)acrylates (e.g., N-morpholinoethyl-acrylate). The preferred co-initiators are aminobenzoates.

The radiation curable inkjet inks preferably includes the (diffusion hindered) co-initiator in an amount of 0.1 to 20 wt%, more preferably in an amount of 0.5 to 15 wt%, most preferably in an amount of 1 to 10 wt% of the total weight of the radiation curable inkjet ink.

### Polymerization Inhibitors

The radiation curable inkjet ink may contain at least one inhibitor for improving the thermal stability of the ink.

Suitable polymerization inhibitors include phenol type antioxidants, hindered amine light stabilizers, phosphor type antioxidants, hydroquinone monomethyl ether commonly used in (meth)acrylate monomers, and hydroquinone. t-butylcatechol, pyrogallol, 2,6-di-tert.butyl-4-methylphenol (=BHT) and phenothiazine may also be used.

Suitable commercial inhibitors are, for example, Sumilizer™ GA-80, Sumilizer™ GM and Sumilizer™ GS produced by Sumitomo Chemical Co. Ltd.; Genorad™ 16, Genorad™18 and Genorad™ 22 from Rahn AG; Irgastab™UV10 and Irgastab™ UV22, Tinuvin™ 460 and CGS20 from Ciba Specialty Chemicals; Florstab™ UV range (UV-1, UV-2, UV-5 and UV-8) from Kromachem Ltd, Additol™ S range (S100, S110, S120 and S130) and PTZ from Cytec Solvay Group.

The inhibitor is preferably a polymerizable inhibitor.

Since excessive addition of these polymerization inhibitors may lower the curing speed, it is preferred that the amount capable of preventing polymerization is determined prior to blending. The amount of a polymerization inhibitor is preferably lower than 5 wt%, more preferably lower than 3 wt% of the total radiation curable inkjet ink.

### Surfactants

The radiation curable inkjet may contain at least one surfactant, but preferably no surfactant is present.

The surfactant can be anionic, cationic, non-ionic, or zwitter-ionic and is usually added in a total quantity less than 1wt% based on the total weight of the radiation curable inkjet ink.

Suitable surfactants include fluorinated surfactants, fatty acid salts, ester salts of a higher alcohol, alkylbenzene sulfonate salts, sulfosuccinate ester salts and phosphate ester salts of a higher alcohol (for example, sodium dodecylbenzenesulfonate and sodium dioctylsulfosuccinate), ethylene oxide adducts of a higher alcohol, ethylene oxide adducts of an alkylphenol, ethylene oxide adducts of a polyhydric alcohol fatty acid ester, and acetylene glycol and ethylene oxide adducts thereof (for example, polyoxyethylene nonylphenyl ether, and SURFYNOL™ 104, 104H, 440, 465 and TG available from AIR PRODUCTS & CHEMICALS INC.).

Preferred surfactants are selected from fluoric surfactants (such as fluorinated hydrocarbons) and silicone surfactants. The silicone surfactants are preferably siloxanes and can be alkoxylated, polyether modified, polyether modified hydroxy functional, amine modified, epoxy modified and other modifications or combinations thereof. Preferred siloxanes are polymeric, for example polydimethylsiloxanes.

Preferred commercial silicone surfactants include BYK™ 333 and BYK™ UV3510 from BYK Chemie and Tego Rad 2100 from Evonik Industries.

In a preferred embodiment, the surfactant is a polymerizable compound.

Preferred polymerizable silicone surfactants include a (meth)acrylated silicone surfactant. Most preferably the (meth)acrylated silicone surfactant is an acrylated silicone surfactant, because acrylates are more reactive than methacrylates.

In a preferred embodiment, the (meth)acrylated silicone surfactant is a polyether modified (meth)acrylated polydimethylsiloxane or a polyester modified (meth)acrylated polydimethylsiloxane.

Preferably the surfactant is present in the radiation curable inkjet ink in an amount of 0 to 3 wt% based on the total weight of the radiation curable inkjet ink.

### Flame retardant

Preferred flame retardants are inorganic flame retardants, such as Alumina Trihydrate and Boehmite, and organo-phosphor compounds, such as organo-phosphates (e.g. triphenyl phosphate (TPP), resorcinol bis (diphenylphosphate) (RDP), bisphenol A diphenyl phosphate (BADP), and tricresyl phosphate (TCP)); organo-phosphonates (e.g. dimethyl methylphosphonate (DMMP)); and organophosphinates (e.g. aluminium dimethylphosphinate).

Other preferred organo-phosphor compounds are disclosed in US8273805.

### Preparation of Inkjet Inks

The preparation of pigmented radiation curable inkjet inks is well-known to the skilled person. Preferred methods of preparation are disclosed in paragraphs [0076] to [0085] of WO2011/069943.

### Manufacture of a Printed Circuit Board

The method of manufacturing a Printed Circuit Board (PCB) according to the present invention includes an inkjet printing step wherein a radiation curable inkjet ink as described above is jetted and cured on a substrate.

According to a preferred embodiment, the method of manufacturing a PCB includes an inkjet printing step wherein an etch resist is provided on a metal surface, preferably a copper surface.

An etch resist is provided on the metal surface by jetting and curing the radiation curable inkjet ink on the metal surface thereby forming a protected area of the metal surface. Metal from an unprotected area of the metal surface is then removed by etching. After etching, at least part of the etch resist is removed from the protected area of the metal surface.

The metal surface is preferably a metal foil or sheet attached to a substrate.

There is no real limitation on the type of substrate bonded to the metal sheet as long as it is non-conductive. The substrates may be made of a ceramic, glass or plastics, such as polyimides.

The metal sheet usually has a thickness between 9 and 105 µm.

There is no limitation on the nature of the metal surface. The metal surfaces preferably consist of copper, aluminium, nickel, iron, tin, titanium or zinc, but may be also alloys including these metals. In a very preferred embodiment, the metal surface is made of copper. Copper has a high electrical conductivity and is a relatively cheap metal, making it very suitable for making printed circuit boards.

The method may also be used for manufacturing a decorative etched metal panel.

The metal surface used may be selected from the metals described above for the embodiment wherein conductive patterns are prepared. In this case, preferably a solid metal panel is used. However, also a metal foil attached to a substrate may be used. There is no real limitation on the type of substrate bonded to the metal foil. The substrates may be made of a ceramic, glass or plastics, or even a second (cheaper) metal plate. The metal may also be an alloy.

Such a decorative metal panel may serve a purpose other than being purely decorative, such as providing information. For example, an aluminium name plate wherein the etch resistant radiation curable inkjet ink was printed as information, such as a name of a person or a company, and then removed to result in a glossy shiny name on a mat etched background, is also considered a decorative metal panel including a decorative element. Etching causes a change in optical properties of a metal surface, such as a change of gloss. After removal of the cured radiation curable inkjet ink from the metal surface an aesthetic effect is created between the etched and the non-etched metal surface.

In a preferred embodiment of the inkjet printing method, the metal surface is cleaned before printing the radiation curable inkjet ink. This is especially desirable when the metal surface is handled by hand and no gloves are worn. The cleaning removes dust particles and grease which can interfere in the adhesion of the radiation curable inkjet ink to the metal surface. In PCB the copper is often cleaned by microetching. The oxide layer of the copper is removed and roughness introduced in order to improve the adhesion.

The inkjet method may also be used for manufacturing a decorative etched glass panel. Such a method is disclosed in for example WO2013/189762 (AGC).

According to another preferred embodiment, the method of manufacturing a PCB comprises an inkjet printing step wherein a solder mask is provided.

The solder mask is provided by jetting and curing the radiation curable inkjet ink typically on a dielectric substrate containing an electrically conductive pattern.

A heat treatment is preferably applied to the jetted and cured radiation curable inkjet ink. The heat treatment is preferably carried out at a temperature between 80°C and 250°C. The temperature is preferably not less than 100°C, more preferably not less than 120°C. To prevent charring of the solder mask, the temperature is preferably not greater than 200°C, more preferably not greater than 160°C.

The thermal treatment is typically carried out between 15 and 90 minutes.

The purpose of the thermal treatment is to further increase the polymerization degree of the solder mask.

The dielectric substrate of the electronic device may be any non-conductive material. The substrate is typically a paper/resin composite or a resin/fibre glass composite, a ceramic substrate, a polyester or a polyimide.

The electrically conductive pattern is typically made from any metal or alloy which is conventionally used for preparing electronic devices such as gold, silver, palladium, nickel/gold, nickel, tin, tin/lead, aluminium, tin/aluminium and copper. The electrically conductive pattern is preferably made from copper.

The radiation curable inkjet ink may be cured in both embodiments by exposing the ink to actinic radiation, such as electron beam or ultraviolet (UV) radiation. Preferably the radiation curable inkjet ink is cured by UV radiation, more preferably using UV LED curing.

The method of manufacturing a PCB may comprise two, three or more inkjet printing steps. For example the method may comprise two inkjet printing steps wherein an etch resist is provided on a metal surface in one inkjet printing step and wherein a solder mask is provided on a dielectric substrate containing an electrically conductive pattern in another inkjet printing step.

A third inkjet printing step may be used for legend printing.

### Etching

Etching of a metal surface is performed by using an etchant. The etchant is preferably an aqueous solution having a pH < 3 or wherein 8 < pH < 10.

In a preferred embodiment, the etchant is an acid aqueous solution having a pH of less than 2. The acid etchant preferably includes at least one acid selected from the group consisting of nitric acid, picric acid, hydrochloric acid, hydrofluoric acid and sulphuric acid.

Preferred etchants known in the art include Kalling's N°2, ASTM N° 30, Kellers Etch, Klemm's Reagent, Kroll's Reagent, Marble's Reagent, Murakami's Reagent, Picral and Vilella's Reagent.

In another preferred embodiment, the etchant is an alkaline aqueous solution having a pH of no more than 9. The alkaline etchant preferably includes at least one base selected from the group consisting of ammonia or ammonium hydroxide, potassium hydroxide and sodium hydroxide.

The etchant may also contain a metal salt such as copper dichloride, copper sulphate, potassium ferricyanide and iron trichloride.

Etching of a metal surface in PCB applications is preferably performed in a time frame of seconds to a few minutes, more preferably 5 to 200 seconds. Etching is preferably performed at a temperature between 35 and 60°C.

The etching time of a metal surface in other applications, such as in the manufacture of decorative metal panels, may be substantially longer, depending on the type and amount of metal that has to be removed during the etch step. Etching times may be more then 15, 30 or even 60 minutes.

In the method wherein a glass surface is etched, the etching solution is preferably an aqueous solution of hydrofluoric acid. Typically, the etching solution has a pH between 0 and 5.

Etching is preferably followed by rinsing with water to remove any residual etchant.

### Stripping

After etching, the cured radiation curable inkjet ink must at least partially be removed from the metal surface, so that e.g. electric or electronic devices can make contact with the remaining metal surface (conductive pattern) or that the decorative feature of an etched metal panel becomes fully visible. For example, an electronic component such as a transistor must be able to make electrical contacts with the conductive (copper) pattern on the printed circuit board. In a preferred embodiment, the cured radiation curable inkjet ink is completely removed from the metal surface.

In a preferred embodiment, the cured radiation curable inkjet ink is removed by an alkaline stripping bath. Such an alkaline stripping bath is usually an aqueous solution with a pH > 10.

In another embodiment, the cured radiation curable inkjet ink is removed by dry delamination. This technique of "dry stripping" is currently unknown in the art of manufacturing printed circuit boards and introduces several ecological and economical advantages in the manufacturing process. Dry stripping not only eliminates the need of a corrosive alkaline stripping bath and its inherent liquid waste, but also allows for a higher throughput. Dry stripping can be implemented, for example, by using an adhesive foil and a roll-to-roll laminator-delaminator. The adhesive foil is first laminated with its adhesive side onto the cured radiation curable inkjet ink present on the metal surface and subsequently delaminated thereby removing the cured radiation curable inkjet ink from the metal surface. Delamination by a roll-to-roll laminator-delaminator can be performed in seconds, while alkaline stripping can take minutes.

### Inkjet Printing Devices

The radiation curable inkjet ink may be jetted by one or more print heads ejecting small droplets in a controlled manner through nozzles onto a substrate, which is moving relative to the print head(s).

A preferred print head for the inkjet printing system is a piezoelectric head. Piezoelectric inkjet printing is based on the movement of a piezoelectric ceramic transducer when a voltage is applied thereto. The application of a voltage changes the shape of the piezoelectric ceramic transducer in the print head creating a void, which is then filled with ink. When the voltage is again removed, the ceramic expands to its original shape, ejecting a drop of ink from the print head. However the inkjet printing method according to the present invention is not restricted to piezoelectric inkjet printing. Other inkjet print heads can be used and include various types, such as a continuous type.

The inkjet print head normally scans back and forth in a transversal direction across the moving ink-receiver surface. Often the inkjet print head does not print on the way back. Bi-directional printing is preferred for obtaining a high areal throughput. Another preferred printing method is by a "single pass printing process", which can be performed by using page wide inkjet print heads or multiple staggered inkjet print heads which cover the entire width of the ink-receiver surface. In a single pass printing process the inkjet print heads usually remain stationary and the ink-receiver surface is transported under the inkjet print heads.

### Curing Devices

The radiation curable inkjet ink can be cured by exposing them to actinic radiation, such as electron beam or ultraviolet radiation. Preferably the radiation curable inkjet ink is cured by ultraviolet radiation, more preferably using UV LED curing.

In inkjet printing, the curing means may be arranged in combination with the print head of the inkjet printer, travelling therewith so that the curable liquid is exposed to curing radiation very shortly after been jetted.

In such an arrangement, with the exception of UV LEDs, it can be difficult to provide a small enough radiation source connected to and travelling with the print head. Therefore, a static fixed radiation source may be employed, e.g. a source of curing UV-light, connected to the radiation source by means of flexible radiation conductive means such as a fibre optic bundle or an internally reflective flexible tube.

Alternatively, the actinic radiation may be supplied from a fixed source to the radiation head by an arrangement of mirrors including a mirror upon the radiation head.

The source of radiation may also be an elongated radiation source extending transversely across the substrate to be cured. It may be adjacent the transverse path of the print head so that the subsequent rows of images formed by the print head are passed, stepwise or continually, beneath that radiation source.

Any ultraviolet light source, as long as part of the emitted light can be absorbed by the photo-initiator or photo-initiator system, may be employed as a radiation source, such as, a high or low pressure mercury lamp, a cold cathode tube, a black light, an ultraviolet LED, an ultraviolet laser, and a flash light. Of these, the preferred source is one exhibiting a relatively long wavelength UV-contribution having a dominant wavelength of 300-400 nm. Specifically, a UV-A light source is preferred due to the reduced light scattering therewith resulting in more efficient interior curing.

UV radiation is generally classed as UV-A, UV-B, and UV-C as follows:
- UV-A: 400 nm to 320 nm
- UV-B: 320 nm to 290 nm
- UV-C: 290 nm to 100 nm.

In a preferred embodiment, the radiation curable inkjet ink is cured by UV LEDs. The inkjet printing device preferably contains one or more UV LEDs preferably with a wavelength larger than 360 nm, preferably one or more UV LEDs with a wavelength larger than 380 nm, and most preferably UV LEDs with a wavelength of about 395 nm.

Furthermore, it is possible to cure the ink image using, consecutively or simultaneously, two light sources of differing wavelength or illuminance. For example, the first UV-source can be selected to be rich in UV-C, in particular in the range of 260 nm-200 nm. The second UV-source can then be rich in UV-A, e.g. a gallium-doped lamp, or a different lamp high in both UV-A and UV-B. The use of two UV-sources has been found to have advantages e.g. a fast curing speed and a high curing degree.

For facilitating curing, the inkjet printing device often includes one or more oxygen depletion units. The oxygen depletion units place a blanket of nitrogen or other relatively inert gas (e.g. CO₂), with adjustable position and adjustable inert gas concentration, in order to reduce the oxygen concentration in the curing environment. Residual oxygen levels are usually maintained as low as 200 ppm, but are generally in the range of 200 ppm to 1200 ppm.

### EXAMPLES

### Materials

All materials used in the following examples were readily available from standard sources such as ALDRICH CHEMICAL Co. (Belgium) and ACROS (Belgium) unless otherwise specified. The water used was deionized water.

**CTFA** is a cyclic trimethylpropane formal acrylate available as Sartomer™ SR531 from ARKEMA.

**VEEA** is 2-(vinylethoxy)ethyl acrylate available from NIPPON SHOKUBAI, Japan.

**SR335** is a laurylacrylate available as Sartomer™ SR335 from ARKEMA.

**ACMO** is acryloyl morpholine available from RAHN.

**CD420** is a monofunctional cyclic acrylic monomer available as Sartomer™ CD420 from ARKEMA.

**TMPTA** is trimethylolpropane triacrylate available as Sartomer™ SR351 from ARKEMA.

**ITX** is Speedcure™ ITX, a mixture of isopropyl thioxanthone isomers, from LAMBSON SPECIALTY CHEMICALS.

**EPD** is ethyl-4-(dimethylamino)benzoate, available under the trade name of Genocure™ EPD from RAHN AG.

**BAPO** is a bis(2,4,6-trimethylbenzoyl)-phenylphosphineoxide photoinitiator available as Irgacure™ 819 from BASF.

**INHIB** is a mixture forming a polymerization inhibitor having a composition according to Table 4.

**Table 4**

| **Component** | **wt%** |
|---|---|
| **DPGDA** | 82.4 |
| **p-methoxyphenol** | 4.0 |
| **2,6-di-tert-butyl-4-methylphenol** | 10.0 |
| **Cupferron™ AL** | 3.6 |

**Cupferron™ AL** is aluminum N-nitrosophenylhydroxylamine from WAKO CHEMICALS LTD.

**DPGDA** is dipropylenediacrylate, available as Sartomer SR508 from ARKEMA.

**Ebecryl 1360** is a polysiloxane hexa acrylate slip agent from ALLNEX.

**Cyan** is SUN FAST BLUE 15:4, a cyan pigment available from SUN CHEMICALS.

**Yellow** is CROMOPHTAL YELLOW D 1085J, a yellow pigment from BASF.

**Disperbyk 162** is a dispersing agent and has been precipitated from a solution available from BYK (ALTANA).

**PB5** is a branched poly(4-hydroxystyrene) available as PB5 from HYDRITE CHEMICAL COMPANY

**FR01** is a flame retardant commercially available under tradename ADK Stab FP600 from ADEKA PALMAROL.

**Acrylic acid** 99,5% from ACROS.

### Methods

### Viscosity

The viscosity of the inks was measured at 45°C and at a shear rate of 1000 s⁻¹ using a "Robotic Viscometer Type VISCObot" from CAMBRIDGE APPLIED SYSTEMS.

For industrial inkjet printing, the viscosity at 45°C and at a shear rate of 1000 s⁻¹ is preferably between 5.0 and 15 mPa.s. More preferably the viscosity at 45°C and at a shear rate of 1 000 s⁻¹ is less than 15 mPa.s.

### Adhesion

The adhesion was evaluated by a cross-cut test according to ISO2409:1992(E). Paints (International standard 1992-08-15) using a Braive No.1536 Cross Cut Tester from BRAIVE INSTRUMENTS with spacing of a 1 mm between cuts and using a weight of 600 g, in combination with a Tesatape™ 4104 PVC tape. The evaluation was made in accordance with a criterion described in Table 5, where both the adhesion in the cross-cut and outside the cross-cut were evaluated.

**Table 5**

| **Evaluation value** | **Criterion** |
|---|---|
| **0** | Nothing removed, perfect adhesion. |
| **1** | Detachment of only very small parts of the cured layer, almost perfect adhesion. |
| **2** | Minor parts of the cured layer was removed by the tape, good adhesion |
| **3** | Parts of the cured layer were removed by the tape, poor adhesion. |
| **4** | Most of the cured layer was removed by the tape, poor adhesion. |
| **5** | The cured layer was completely removed from the substrate by the tape, no adhesion. |

### Solder resistance

The solder resistance of the solder mask inkjet inks was evaluated using a SPL600240 Digital Dynamic Solder Pot available from L&M PRODUCTS filled with a "K" Grade 63:37 tin/lead solder available from SOLDER CONNECTION. The temperature of the solder was set at 290°C.

Using a Q-tip, a solder flux SC7560A from SOLDER CONNECTION was applied on the surface of the samples (i.e. coatings of the solder mask inkjet ink on a copper surface as described under adhesion) to clean the surface. The solder flux was dried by placing the samples for 10 minutes above the solder pot.

After placing the sample in the solder pot, a solder wave was created for 10 seconds after which the samples were cooled for at least 10 minutes.

The adhesion of the solder mask inkjet inks on the copper surface was then evaluated with a tape test on the cooled samples. A black tape Tesa 4104/04 from TESA AG, Germany was taped onto the coating and the tape was removed immediately thereafter by hand.

A visual evaluation resulted in an adhesion quality ranging from 0 (very good adhesion) to 5 (very poor adhesion).

### Preparation of the photoinitiators

The photoinitiators INI-1 to INI-5 and INI-15 were prepared as follows.

### Materials

[chloro(phenyl)phosphoryl]-(2,4,6-trimethylphenyl)methanone (CASRN577956-23-2) was prepared as disclosed in DE10206117.

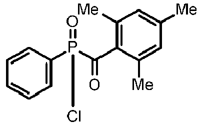

2-[phenyl-(2,4,6-trimethylbenzoyl)phosphoryl]oxyethyl prop-2-enoate (CASRN578738-30-4) was prepared as disclosed in DE10206096.

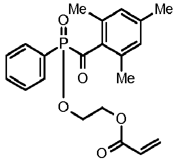

2-[4-(2-hydroxy-2-methyl-propanoyl)phenoxy]ethyl prop-2-enoate (CASRN110430-09-6) was prepared as disclosed in WO2010/069758.

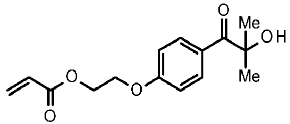

2-[[2-(9-oxothioxanthen-2-yl)oxyacetyl]-(2-prop-2-enoyloxyethyl)amino]ethyl prop-2-enoate was prepared according to WO2009030658.

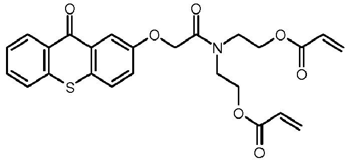

### LC-MS analysis

Some photoinitiators were analyzed on an AmaZon™ SL mass spectrometer (supplied by Brüker Daltonics), using an Alltech Alltima C18 (150 mm x 3.2 mm) column at a flow rate of 0.5 ml/min at a temperature of 30°C and ESI as ionisation technique. A gradient elution was used as described below :
- Eluent A : 10 mmol ammonium acetate in water/methanol 9/1
- Eluent B : 10 mmol ammonium acetate in methanol

| Elution time (min) | % eluent B |
|---|---|
| 0 | 0 |
| 15 | 100 |
| 21 | 100 |
| 22 | 0 |
| 30 | Stop |

### The synthesis of INI-1

The photoinitiator INI-1 was prepared according to the following reaction scheme.

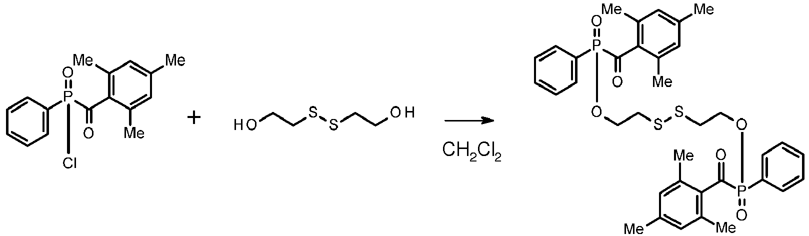

15.33 g (0.05 mol) [chloro(phenyl)phosphoryl]-(2,4,6-trimethylphenyl)methanone was dissolved in 60 ml methylene chloride. A solution of 3.08 g (0.02 mol) 2,2'-hydroxyethyl disulfide in 15 ml methylene chloride was added over five minutes.

The reaction was allowed to continue for 16 hours at room temperature.

An additional 6.1 g (0.01 mol) [chloro(phenyl)phosphoryl]-(2,4,6-trimethylphenyl)-methanone was added and the reaction mixture was refluxed for 24 hours.

The reaction mixture was allowed to cool down to room temperature and the solvent was removed under reduced pressure.

INI-1 was purified by preparative column chromatography, on a Prochrom LC80 column, using Kromasil Si60 10µm as stationary phase and methylene chloride/ethyl acetate 60/40 as eluent.

4.1 g of INI-1 was isolated (yield = 29.5%) (TLC analysis on TLC Silica gel 60F254 supplied by Merck, eluent n.-hexane/ethyl acetate 60/40, Rf : 0.5).

INI-1 was analyzed using ¹H-NMR-spectroscopy (DMSO d6) (see Table 6).

**Table 6**

| | | |
|---|---|---|
| 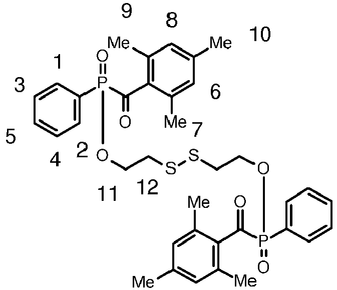 | 1,2,5 | 7.76 ppm (m, 6H) |
| | 3,4 | 7.59 ppm (m, 4H) |
| | 7,9 | 2.08 ppm (s, 12 H) |
| | 8,6 | 6.90 ppm (s, 4H) |
| | 10 | 2.23 ppm (s, 6H) |
| | 11 | 4.15 ppm (m, 4H) |
| | 12 | 2.92 ppm (t, 4H) |

### The synthesis of INI-2

The photoinitiator INI-2 was prepared according to the following reaction scheme.

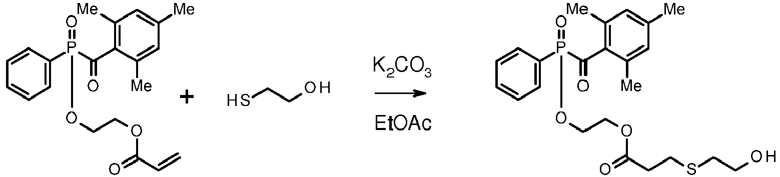

11.97 g (0.031 mol) 2-[phenyl-(2,4,6-trimethylbenzoyl)phosphoryl]oxyethyl prop-2-enoate was dissolved in 160 ml ethyl acetate. 0.136 g BHT was added, followed by the addition of 4.28 g (0.031 mol) potassium carbonate. 2.66 g (0.034 mol) 2-mercapto-ethanol was added followed by the addition of 40 ml ethyl acetate.

The reaction was allowed to continue at 75°C for three hours.

The reaction mixture was allowed to cool down to room temperature, the potassium carbonate was removed by filtration and the solvent was evaporated under reduced pressure.

INI-2 was purified by preparative column chromatography on a Graceresolve RS80 column using a gradient elution from methylene chloride to ethyl acetate.

4 g of INI-2 was isolated (yield = 28 %) (TLC on analysis on TLC Silica gel 60F254 supplied by Merck, eluent methylene chloride/ethyl acetate 60/40 : Rf : 0.35).

INI-2 was analyzed using LC-MS, according to the method described above. The molecular mass of the structure was confirmed and the purity was 99.1 area%.

### The synthesis of INI-3

The photoinitiator INI-3 was prepared according to the following reaction scheme.

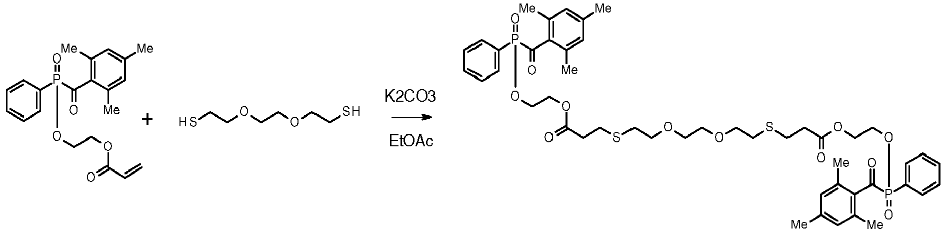

20.28 g (0.053 mol) 2-[phenyl-(2,4,6-trimethylbenzoyl)phosphoryl]oxyethyl prop-2-enoate was dissolved in 200 ml ethyl acetate. 0.57 g BHT and 3.45 g (0.025 g) potassium carbonate were added. 4.55 g (0.025 mol) 1,8-dimercapto-3,6-dioxaoctane was added, followed by the addition of 50 ml ethyl acetate.

The mixture was refluxed for 3 hours.

The reaction mixture was allowed to cool down to room temperature.

Potassium carbonate was removed by filtration and the solvent was evaporated under reduced pressure.

INI-3 was purified by preparative column chromatography on a Graceresolve RS80 column using a gradient elution from methylene chloride to ethyl acetate. 13.4 g of INI-3 was isolated (yield = 56%) (TLC analysis on TLC Silica gel 60F₂₅₄ supplied by Merck, eluent ethyl acetate : R_{f} : 0.5)

INI-3 was analyzed using ¹H-NMR-spectroscopy (DMSO d6) (see Table 7).

**Table 7**

| | | |
|---|---|---|
| 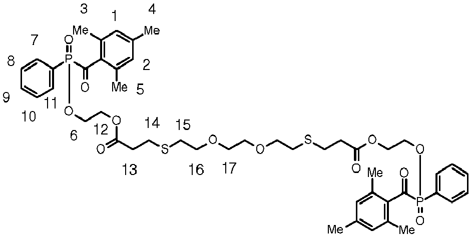 | 1,2 | 6.90 pp (s, 4H) |
| | 3,5 | 2.08 ppm (s, 12 H) |
| | 4 | 2.25 ppm (s, 6H) |
| | 6, 12 | 4.19 ppm (m, 8 H) |
| | 7,9,11 | 7.77 ppm (m, 6H) |
| | 8,10 | 7.61 ppm (m, 4H) |
| | 13 | 2.51 ppm (t, 4H) |
| | 14 | 2.67 ppm (t, 4H) |
| | 15 | 2.64 ppm (t, 4H) |
| | 16 | 3.55 ppm (t, 4H) |
| | 17 | 3.47 ppm (s, 4H) |

### The synthesis of INI-4

The photoinitiator INI-4 was prepared according to the following reaction scheme.

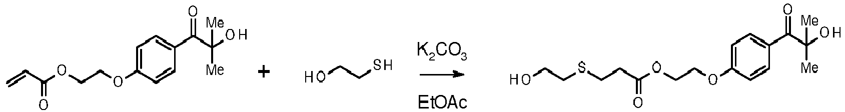

27.8 g (0.1 mol) 2-[4-(2-hydroxy-2-methyl-propanoyl)phenoxy]ethyl prop-2-enoate was dissolved in 280 ml ethyl acetate. 0.44 g BHT and 6.9 g (0.05 mol) potassium carbonate were added. 8.6 g (0.11 mol) 2-mercapto-ethanol was added followed by the addition of 20 ml ethyl acetate.

The reaction mixture was refluxed for two hours.

The reaction mixture was allowed to cool down to room temperature, the potassium carbonate was removed by filtration and the solvent was evaporated under reduced pressure.

INI-4 was purified by preparative column chromatography on a Graceresolve RS80 column using a gradient elution from methylene chloride to ethyl acetate. 20.2 g of INI-4 was isolated (yield = 58%) (TLC analysis on TLC Silica gel 60F₂₅₄ supplied by Merck, eluent n.-hexane/ethyl acetate 25/75 : R_{f} : 0.35). INI-4 was analyzed using ¹H-NMR-spectroscopy (DMSO d6) (see Table 8).

**Table 8**

| | | |
|---|---|---|
| 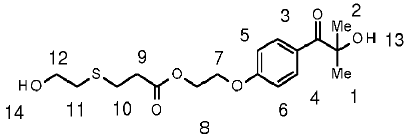 | 1,2 | 1.39 ppm (6H) |
| | 3,4 | 8.11 ppm (d, 2H) |
| | 5,6 | 7.02 ppm (d, 2H) |
| | 7 | 4.31 ppm (t, 2H) |
| | 8 | 4.38 ppm (t, 2H) |
| | 9 | 2.68 ppm (t, 2H) |
| | 10 | 2.72 ppm (t, 2H) |
| | 11 | 2.56 ppm(t, 2 H) |
| | 12 | 3.53 ppm (m, 2H) |
| | 13 | 5.67 ppm (s, 1H) |
| | 14 | 4.78 ppm (t, 1H) |

### The synthesis of INI-5

The photoinitiator INI-5 was prepared according to the following reaction scheme.

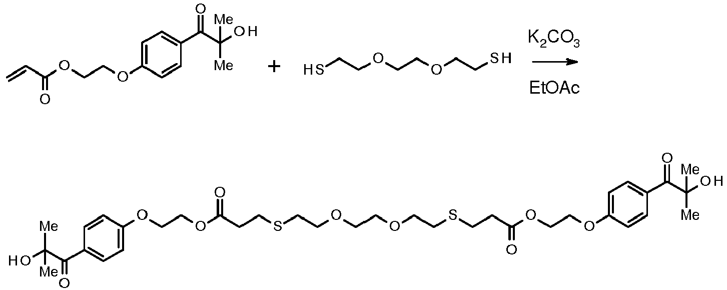

13.91 g (0.05 mol) 2-[4-(2-hydroxy-2-methyl-propanoyl)phenoxy]ethyl prop-2-enoate was dissolved in 200 ml ethyl acetate. 0.11 g BHT and 1.72 g (0.0125 mol) potassium carbonate were added, followed by the addition of 4.55 g (0.025 mol) 1,8-dimercapto-3,6-dioxaoctane and 50 ml ethyl acetate.

The reaction mixture was refluxed for 3 hours.

The reaction mixture was allowed to cool down to room temperature, potassium carbonate was removed by filtration and the solvent was evaporated under reduced pressure.

INI-5 was purified by preparative column chromatography on a Prochrom LC80 column, using Kromasil Si60 10µm as stationary phase using a gradient elution from methylene chloride/ethyl acetate 70/30 to methylene chloride/ethyl acetate 50/50. 12 g of INI-5 was isolated (yield = 65 %) (TLC analysis on TLC Silica gel 60F254 supplied by Merck, eluent methylene chloride/ethyl acetate 40/60 : Rf : 0.5).

INI-5 was analyzed using LC-MS, according to the method described above. The molecular mass of the structure was confirmed and the purity was 95.8 area%. The following structure was identified as contaminant.

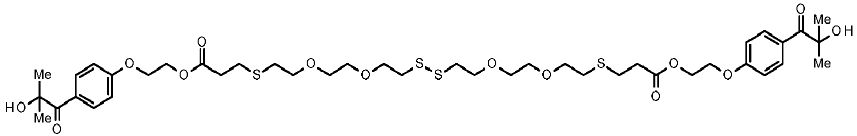

### The synthesis of INI-15

The photoinitiator INI-5 was prepared according to the following reaction scheme.

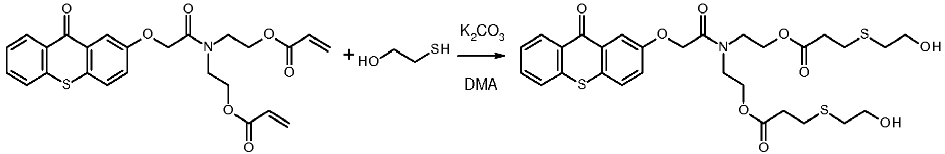

15 g (31 mmol) 2-[[2-(9-oxothioxanthen-2-yl)oxyacetyl]-(2-prop-2-enoyloxyethyl)-amino]ethyl prop-2-enoate was dissolved in 180 ml dimethyl acetamide. 0.27 g BHT and 4.28 g (31 mmol) potassium carbonate were added. A solution of 5.35 g (68.5 mmol) 2-mercapto-ethanol in 20 ml dimethyl acetamide was added and the reaction was allowed to continue at 70°C for 90 minutes.

The reaction mixture was allowed to cool down to room temperature.

The potassium carbonate was removed by filtration and the solvent was evaporated under reduced pressure.

INI-15 was purified by preparative column chromatography on a Prochrom LC80 column, using Kromasil Si60 10µm as stationary phase and methylene chloride/methanol 90/10 as eluent. 9 g of INI-15 was isolated (yield = 45.5 %). (TLC analysis on TLC Silica gel 60F₂₅₄ supplied by Merck, eluent methylene chloride/methanol 95/5 : R_{f} = 0.24).

INI-15 was analyzed using LC-MS, according to the method described above. INI-15 was a mixture of three isomeric structures with 88 area% of the structure according to the reaction scheme.

### Green pigment dispersion GPD

A concentrated Green pigment dispersions, GPD, was prepared having a composition according to Table 9.

GPD was prepared as follows: 138 g of 2-(2-vinyloxyethoxy)ethyl acrylate, 2 g of a solution containing 4 wt% of 4-methoxyphenol, 10 wt% of 2,6-di-tert-butyl-4-methylphenol and 3.6 wt% of Aluminum-N-nitroso phenylhydroxyl amine in dipropylene glycol diacrylate and 30 g of Cyan and 30 g of Yellow were mixed using a DISPERLUX™ dispenser. Stirring was continued for 30 minutes. The vessel was connected to a NETZCH MiniZeta mill filled with 900 g of 0.4 mm yttrium stabilized zirconia beads ("high wear resistant zirconia grinding media" from TOSOH Co.). The mixture was circulated over the mill over 120 minutes (residence time of 45 minutes) and a rotation speed in the mill of about 10.4 m/s. During the complete milling procedure the content in the mill was cooled to keep the temperature below 60°C. After milling, the dispersion was discharged into a vessel.

**Table 9**

| GPD | wt% |
|---|---|
| Cyan | 7.5 |
| Yellow | 7.5 |
| Disperbyk 162 | 15 |
| AGFARAD | 1 |
| VEEA | 69 |

### Example 1

This example illustrates the effect of the photoinitiators according to the present invention.

### Preparation of the inventive and comparative inkjet inks INV-01 to INV-05 and COMP-01

The comparative radiation curable inkjet ink COMP-01 and the inventive radiation curable inkjet inks INV-01 to INV-05 were prepared according to Table 10. The weight percentages (wt%) are all based on the total weight of the radiation curable inkjet ink.

**Table 10**

| **wt% of component** | COMP-01 | INV-01 | INV-02 | INV-03 | INV-04 | INV-05 |
|---|---|---|---|---|---|---|
| **GPD** | 6.60 | = | = | = | = | = |
| **CTFA** | 20.00 | = | = | = | = | = |
| **VEEA** | 24.17 | 21.37 | = | = | = | = |
| **SR335** | 5.00 | = | = | = | = | = |
| **ACMO** | 5.00 | = | = | = | = | = |
| **CD420** | 15.00 | = | = | = | = | = |
| **TMPTA** | 5.00 | = | = | = | = | = |
| **PB5** | 5.00 | = | = | = | = | = |
| **FR01** | 2.00 | = | = | = | = | = |
| **ITX** | 4.00 | = | = | = | = | = |
| **EPD** | 4.00 | = | = | = | = | = |
| **BAPO** | 2.00 | = | = | = | = | = |
| **Acrylic acid** | 1.20 | - | - | - | - | - |
| **INI-2** | - | 4.00 | - | - | - | - |
| **INI-3** | - | - | 4.00 | - | - | - |
| **INI-4** | - | - | - | 4.00 | - | - |
| **INI-5** | - | - | - | - | 4.00 | - |
| **INI-15** | - | - | - | - | - | 4.00 |
| **Ebecryl 1360** | 0.10 | = | = | = | = | = |
| **INHIB** | 0.93 | = | = | = | = | = |

The comparative sample COMP-1 and the inventive samples INV-1 to INV-04 were obtained by jetting the inks on a 35 µm brushed Cu laminate or a brushed FR laminate using an Anapurna M2050i (8 pass, 45°C jetting temperature, 100 % pincure after each pass using a LED 395 nm lamp). Additionally a thermal cure at 150°C during 60 minutes was performed.

The solder resistance of the Comparative ink COMP-01 and the inventive inks INV-01 to INV-05 were tested as described above. The results are shown in Table 11.

**Table 11**

| **UV curable ink jet ink** | **Adhesion** | | **Solder Resistance** | |
|---|---|---|---|---|
| | Brushed Cu | Brushed FR4 | Brushed Cu | Brushed FR4 |
| **COMP-1** | 0 | 0 | 0 | 0 |
| **INV-1** | 0 | 0 | 0 | 0 |
| **INV-2** | 0 | 0 | 0 | 0 |
| **INV-3** | 0 | 0 | 0 | 0 |
| **INV-4** | 0 | 0 | 0 | 0 |
| **INV-5** | 2 | 0 | 0 | 0 |

It is clear from the results of Table 11 that the inventive inkjet inks containing a photoinitiator according to the present invention all have a comparable adhesion and solder resistance towards a Cu and a FR surface compared to an inkjet ink comprising an acidic adhesion promotor.

## Claims

1. A radiation curable inkjet ink comprising a polymerizable compound and a photoinitiator, **characterized in that** the photoinitiator comprises a functional group selected from the group consisting of an aliphatic thio-ether and an aliphatic or a (hetero)aromatic disulfide.

2. The radiation curable inkjet ink according to claim 1 wherein the photoinitiator is selected from the group consisting of a thioxanthone, an α-hydroxyketone, an α-aminoketone, an acylphosphine oxide and a bisacyl phosphine oxide.

3. The radiation curable inkjet ink according to claims 1 or 2 wherein the photoinitiator has a chemical structure according to Formula I, 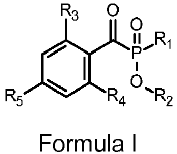 wherein
R₁ is selected from the group consisting of a substituted or unsubstituted aryl group, a substituted or unsubstituted heteroaryl group and an acyl group;
R₂ represents a structural moiety comprising a functional group selected from the group consisting of an aliphatic thio-ether and a disulfide;
R₃ and R₄ are independently selected from the group consisting of a substituted or unsubstituted alkyl group, a substituted or unsubstituted alkoxy group and a halogen; R₅ is selected from the group consisting of a hydrogen, a substituted or unsubstituted alkyl group, a substituted or unsubstituted alkoxy group and a halogen.

4. The radiation curable inkjet ink according to any of the preceding claims wherein the photoinitiator has a chemical structure according to Formula II, 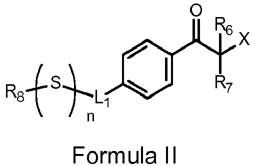 wherein
X is selected from the group consisting of OH and NR₉R₁₀;
R₆ and R₇ are independently selected from the group consisting of a substituted or unsubstituted alkyl group, a substituted or unsubstituted alkenyl group, a substituted or unsubstituted alkynyl group, a substituted or unsubstituted alkaryl group, a substituted or unsubstituted aralkyl group and a substituted or unsubstituted (hetero)aryl group;
R₆ and R₇ may represent the necessary atoms to form a five to eight membered ring; R₈ is selected from the group consisting of a substituted or unsubstituted alkyl group, a substituted or unsubstituted alkenyl group, a substituted or unsubstituted alkynyl group and a substituted or unsubstituted aralkyl group;
L₁ is a divalent linking group having no more than 20 carbon atoms;
R₉ and R₁₀ are independently selected from the group consisting of a substituted or unsubstituted alkyl group, a substituted or unsubstituted alkenyl group, a substituted or unsubstituted alkynyl group, a substituted or unsubstituted alkaryl group, a substituted or unsubstituted aralkyl group and a substituted or unsubstituted (hetero)aryl group;
R₉ and R₁₀ may represent the necessary atoms to form a five to eight membered ring n represents 1 or 2.

5. The radiation curable inkjet ink according to any of the preceding claims wherein the photoinitiator has a chemical structure according to Formula III, 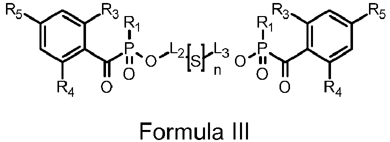 wherein
R₁, R₃, R₄ and R₅ are defined in claim 3;
L₂ and L₃ independently represent a divalent linking groups having no more than 20 carbon atoms;
n represents 1 or 2.

6. The radiation curable inkjet ink according to any of the preceding claims wherein the photoinitiator has a chemical structure according to Formula IV, 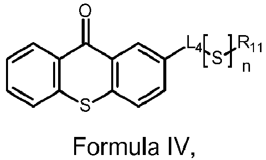 wherein
L₄ is a divalent linking group having no more than 20 carbon atoms;
R₁₁ is selected from the group consisting of a substituted or unsubstituted alkyl group, a substituted or unsubstituted alkenyl group, a substituted or unsubstituted alkynyl group and a substituted or unsubstituted aralkyl group;
n represents 1 or 2.

7. The radiation curable inkjet ink according to any of the preceding claims wherein the polymerizable compound is selected from the group consisting of neopentyl glycol hydroxypivalate diacrylate, isobornyl acrylate, dipropylene glycol diacrylate, trimethylol propane triacrylate, and 2-(vinylethoxy)ethyl acrylate.

8. The radiation curable inkjet ink according to any of the preceding claims further comprising a phenolic resin or a hydroxystyrene based resin.

9. The radiation curable inkjet ink according to any of the preceding claims further comprising a colorant.

10. The radiation curable inkjet ink according to any of the preceding claims further comprising a flame retardant.

11. A method of manufacturing a Printed Circuit Board (PCB) including an inkjet printing step wherein a radiation curable inkjet ink as defined in any of the claims 1 to 10 is jetted and cured on a substrate.

12. The method according to claim 11 wherein curing is carried out using UV radiation.

13. The method according to claim 11 or 12 wherein an etch resist is provided on a metal surface in the inkjet printing step.

14. The method according to any of the claims wherein 11 to 13 wherein a solder mask is provided in the inkjet printing step.

15. The method according to claim 14 also comprising a heating step.
